# DEMANDE DE BREVET EUROPEEN

(11) **EP 0 601 914 A1**
(43) Date de publication de la demande: **15.06.1994**
(21) Numéro de dépôt: 93402909.1
(22) Date de dépôt: 01.12.1993
(51) Int. Cl.: A61K 31/55

(54) **Compositions pharmaceutiques contenant du diltiazem et de la molsidomine**

(30) Priorité: 07.12.1992 FR 9214689
(71) Demandeur: SYNTHELABO, F-92350 Le Plessis Robinson (FR)
(72) Inventeur: Guérin, Hervé, F-92200 Neuilly (FR); Bennet, Ian, Essex CM4-9-LR (GB)
(74) Mandataire: Thouret-Lemaitre, Elisabeth

(57) **Abrégé**

Composition pharmaceutique contenant du diltiazem sous la forme de base ou d'un sel et de la molsidomine.

## Description

La présente invention a pour objet des compositions pharmaceutiques contenant du diltiazem et de la molsidomine.

L'angine de poitrine quelque soit sa forme (angor d'effort, angor spastique, angor mixte, ischémie silencieuse) est une maladie dont le mécanisme physiopathologique fondamental est bien connu. En effet, il y a angor lorsque l'apport en oxygène par les artères coronariennes est insuffisant par rapport à la consommation c'est-à-dire, le besoin, la demande en oxygène du muscle cardiaque. Ainsi l'anti-angoreux idéal devrait augmenter l'apport en oxygène du myocarde et en même temps en diminuer les besoins.

Bien que l'augmentation de l'apport en oxygène ne peut se faire que par une vasodilatation coronarienne, la diminution des besoins fait appel à des mécanismes plus complexes qui comportent la diminution de la précharge, la diminution de la postcharge, et une neutralité, voire diminution de la fréquence cardiaque.

Le diltiazem est un inhibiteur calcique reconnu comme le produit de référence de la classe III des antagonistes calciques de la classification de l'OMS. Il augmente l'apport en oxygène du myocarde par une vasodilatation des muscles vasculaires lisses dans la paroi des artères coronariennes. Plus précisément il freine l'entrée du calcium dans les cellules contractiles et empêche ainsi chaque vasoconstriction intempestive. Il diminue également la consommation en oxygène du myocarde, ceci d'une part par une diminution de la postcharge due à une vasodilatation des muscles vasculaires lisses dans les artères périphériques, et d'autre part par une action légèrement bradycardisante. Cependant le diltiazem n'influence pas la précharge, dernier facteur important dans la diminution de la consommation en oxygène.

La molsidomine est le premier représentant d'une nouvelle famille d'anti-angineux, les sydnonimines.

Elle augmente l'apport en oxygène du myocarde par une vasodilatation des muscles vasculaires lisses dans la paroi des artères coronariennes. Plus précisément, la molsidomine, métabolisée en SIN 1, est un donneur direct de NO qui, puisque le NO correspond vraisemblablement à l'EDRF (endothelial derived relaxing factor), dilate de ce fait activement les artères coronariennes. La molsidomine diminue également la consommation en oxygène du myocarde par une diminution de la précharge. Elle n'influence quasiment pas la postcharge, ni la fréquence cardiaque bien que cette dernière a parfois tendance à augmenter ce qui représente un léger effet délétère.

Les mécanismes d'action des deux produits se complètent aux différents niveaux où une intervention pharmacologique dans l'angor est souhaitable pour :
1) obtenir une augmentation de l'apport en oxygène : Bien que les deux produits provoquent chacun cet effet, le diltiazem freine le volet vasoconstriction inadaptée alors que la molsidomine agit favorablement sur le volet vasodilatation. Par conséquent, il y a une complémentarité des deux quant à leur action sur les artères coronariennes.
2) obtenir une diminution des besoins en oxygène : Le diltiazem, en diminuant la postcharge et la molsidomine en diminuant la précharge, ont chacun une action bénéfique où l'autre produit n'agit pas. Qui plus est, le diltiazem provoque une légère baisse de la fréquence cardiaque et empêche ainsi chaque éventuelle augmentation que pourrait engendrer la molsidomine.

Les compositions pharmaceutiques de l'invention contiennent du diltiazem, sous forme de base ou d'un sel, tel que le chlorhydrate, le malate ou le résinate, de la molsidomine et des excipients appropriés.

Elles peuvent contenir par exemple de 30 à 360 mg de chlorhydrate de diltiazem et de 1 à 12 mg de molsidomine.

La posologie quotidienne peut aller de 90 à 360 mg de chlorhydrate de diltiazem et de 3 à 12 mg de molsidomine.

Les compositions selon l'invention ont une bonne efficacité et permettent une amélioration de la tolérance à l'effort.

Elles peuvent être utilisées dans le traitement des maladies ischémiques cardiaques.

Les compositions de l'invention peuvent être présentées sous toute forme galénique appropriée par exemple sous forme de comprimé ou de gélule, éventuellement sous une forme galénique à libération prolongée.

Les exemples suivants illustrent des formulations de compositions pharmaceutiques selon l'invention.

### Exemple 1 : comprimé à libération immédiate.

| | |
|---|---|
| Diltiazem HCl | 30 mg |
| Molsidomine | 1 mg |
| Lactose | 72,5 mg |
| Polyvidone | 5 mg |
| Stéarate de magnésium | 1,5 mg |
| pour 1 comprimé de | 110 mg |

### Exemple 2 : comprimé à libération modifiée

| | |
|---|---|
| Diltiazem HCl | 60 mg |
| Molsidomine | 2 mg |
| Lactose | 123,5 mg |
| Huile de ricin hydrogénée | 28 mg |
| Polyéthylèneglycol 6000 | 5 mg |
| Stéarate de magnésium | 1,5 mg |
| pour 1 comprimé de | 220 mg |

### Exemple 3 : gélule comportant des grains à libération prolongée.

| | |
|---|---|
| Diltiazem HCl | 300 mg |
| Molsidomine | 6 mg |
| Cellulose microcristalline | 65,2 mg |
| Carboxyméthylcellulose sodique | 3,8 mg |
| Copolymère acrylique | 10,1 mg |
| Ethylcellulose | 8,3 mg |
| Monoglycérides diacétylés | 1,9 mg |
| Stéarate de magnésium | 0,4 mg |
| pour une gélule à | 395,7 mg |

## Revendications

1. Composition pharmaceutique contenant du diltiazem sous la forme de base ou d'un sel et de la molsidomine.

2. Composition pharmaceutique selon la revendication 1 dans laquelle le diltiazem est sous forme de chlorhydrate, de malate ou de résinate.

3. Composition pharmaceutique contenant de 30 à 360 mg de chlorhydrate de diltiazem et de 1 à 12 mg de molsidomine.

4. Composition pharmaceutique selon l'une des revendications 1, 2 ou 3, présentée sous toute forme galénique appropriée dans laquelle le diltiazem et la molsidomine sont associés à des excipients appropriés.
